Europäisches Patentamt

(19) **European Patent Office**

Office européen des brevets

(11) Veröffentlichungsnummer: **0 060 499**
**A2**

(12) **EUROPÄISCHE PATENTANMELDUNG**

(21) Anmeldenummer: 82101888.4

(51) Int. Cl.³: **A 61 K 39/00**, A 61 K 37/02

(22) Anmeldetag: 10.03.82

(30) Priorität: 13.03.81 DE 3109696

(71) Anmelder: **Hunsmann, Gerhard, Dr.-med., Colombistrasse 11, D-7800 Freiburg (DE)**

(43) Veröffentlichungstag der Anmeldung: 22.09.82 Patentblatt 82/38

(72) Erfinder: **Hunsmann, Gerhard, Dr.-med., Colombistrasse 11, D-7800 Freiburg (DE)**

(74) Vertreter: **Rackette, Karl, Dipl.-Phys. Dr.-Ing, Kaiser-Joseph-Strasse 179 Postfach 1310, D-7800 Freiburg (DE)**

(84) Benannte Vertragsstaaten: **AT BE CH DE FR GB IT LI NL SE**

(54) Verfahren zur Herstellung eines als Impfstoff geeigneten synthetischen Peptids und es enthaltender Impfstoff.

(57) Es wird ein Verfahren zur Herstellung eines als Impfstoff geeigneten synthetischen Peptids und ein dieses Peptid enthaltender Impfstoff beschrieben. Das synthetische Peptid wird dadurch hergestellt, daß man die relevante antigene Determinante einer Polypeptidkette eines pathogenen Keimes ermittelt, diese Determinante durch Fragmentierung und Fraktionierung gewinnt, ihre Aminosäuresequenz aufschlüsselt und ein diese Aminosäuresequenz aufweisendes synthetisches Peptid herstellt, das dann als Impfstoff verwendet werden kann.

EP 0 060 499 A2

## Verfahren zur Herstellung eines als Impfstoff geeigneten synthetischen Peptids und es enthaltender Impfstoff

Gegenstand der Erfindung ist ein Verfahren zur Herstellung eines als Impfstoff geeigneten synthetischen Peptids sowie ein Impfstoff, der dieses synthetische Peptid als Wirkstoff enthält.

Die zur Immunprophylaxe und in Ausnahmefällen auch zur Therapie von durch Mikroorganismen hervorgerufenen zur Immunisierung eingesetzten Präparate enthalten entweder lebende, durch Mutation in ihrer Pathogenität abgeschwächte Erreger (Lebendimpfstoffe) oder abgetötete pathogene Keime (Totimpfstoffe) oder deren Gifte. Als Untergruppe der Totimpfstoffe oder Totvakzine sind die Spaltvakzine (subunit vaccines) anzusehen. Bei ihnen wird zur Induktion einer Immunität ein Strukturbestandteil oder ein Produkt eines Erregers, beispielsweise ein Toxin, verwendet.

Lebendimpfstoffe werden am Menschen zur Schutzimpfung gegen Pocken, Poliomyelitis, Masern, Röteln, Gelbfieber, Mumps, Influenza und Tuberkulose eingesetzt. Totimpfstoffe werden gegen Diphterie, Tetanus, Keuchhusten, Typhus und Paratyphus sowie gegen Poliomyelitis und Masern angewandt. In den letzten Jahren wird jedoch an der Herstellung von Spaltvakzinen gearbeitet, die die für die Immunisierung notwendigen Teilkomponenten in angereicherter Form, aber kein genetisches Material enthalten.

Für wenigstens eine Virusgruppe ist bereits ein Spaltvakzin eingeführt, dessen Herstellung allerdings relativ aufwendig ist, denn es müssen pathogene Keime in großen Mengen hergestellt werden, da sich Totimpfstoffe einerseits im menschlichen Körper nicht mehr vermehren, und zum anderen die Präparation der immunogenen Teilkomponenten sehr verlustreich ist.

Die Lebendimpfstoffe induzieren im allgemeinen einen guten Schutz und sind, wenn ein entsprechend attenuierter, d.h. in seiner Pathogenität abgeschwächter Erreger gezüchtet wurde, relativ einfach herzustellen. Unsicherheiten ergeben sich jedoch durch den Impfstoff kontaminierende Viren, einen Rest an Pathogenität, eine Rückmutation der attenuierten Erreger zur vollen Pathogenität, eine unerwünschte Übertragung auf Kontaktpersonen, eine Virusinterferenz sowie die begrenzte Haltbarkeit des Impfstoffs bei Transport und Lagerung.

Totimpfstoffe müssen gewöhnlich mehrfach verabreicht werden, um einen ausreichenden Schutz zu vermitteln, sind aber - eine vollständige Inaktivierung vorausgesetzt - sicherer als Lebendimpfstoffe, da sie nicht die Nebenwirkungen und Nachteile biologisch aktiver Erreger aufweisen. Bei den Totimpfstoffen besteht aber die Gefahr einer Sensibilisierung gegen kontaminierende Kulturbestandteile, beispielsweise Hühnereiweiß. Während auf der einen Seite ihre Herstellung aufwendiger ist, lassen sich Totimpfstoffe leichter transportieren und lagern als Lebendimpfstoffe.

Der Hauptnachteil der heute erhältlichen Impfstoffe besteht darin, daß neben den für die Auslösung des immunologischen Schutzes wesentlichen Strukturen Bestandteile pathogener Organismen in den menschlichen Körper gelangen, die nicht nur verhältnismäßig häufig zu unangenehmen Nebenwirkungen führen, sondern, wenn auch wesentlich seltener, schwere Erkrankungen hervorrufen können (Impfrisiko). Darüberhinaus ist die Herstellung wegen der Züchtung großer Mengen pathogener Keime und der Gewinnung bestimmter Komponenten dieser Keime äußerst aufwendig.

Die Aufgabe der vorliegenden Erfindung besteht nun darin, ein Verfahren zur Herstellung eines als Impfstoff geeigneten Peptids und einen dieses synthetische Peptid als Wirkstoff enthaltenden Impfstoff anzugeben, welches synthetische Peptid sich wesentlich einfacher und problemloser herstellen läßt als die herkömmlichen Impfstoffe, nicht deren Nachteile aufweist und dazu geeignet ist, die Spezifität der Immunisierung wesentlich zu steigern.

Diese Aufgabe wird nun gelöst durch die kennzeichnenden Merkmale des Verfahrens gemäß Hauptanspruch und des Impfstoffs gemäß Anspruch 12.

Die Unteransprüche betreffen besonders bevorzugte Ausführungsformen dieses Erfindungsgegenstandes.

Die vorliegende Erfindung beruht auf der Erkenntnis, daß die Polypeptidketten eines Krankheitserregers bzw. Antigens bestimmte, an der Oberfläche vorliegende Teilsequenzen aufweisen, die als für den Krankheitserreger bzw. das Antigen relevante antigene Determinanten bezeichnet werden und die nach der Verabreichung an einen Organismus zu einer Immunisierung des Organismus gegen den Krankheitserreger bzw. das Antigen führen. Diese relevanten antigenen Determinanten, die eine Folge von etwa 10 bis 30 Aminosäuren umfassen, liegen auf der Primärstruktur des Polypeptids unmittelbar hintereinander vor (kontinuierliche Determinanten) oder bestehen aus zwei Bereichen der Aminosäurekette, die durch sekundäre Faltung in enger räumlicher Nachbarschaft vorliegen (diskontinuierliche Determinanten). Diese antigenen Determinanten, die eine sehr kleine Fraktion des Krankheitserregers bzw. Antigens darstellen, können in ihrer Aminosäuresequenz aufgeschlüsselt werden, so daß es möglich wird, synthetische Peptide gleicher Aminosäuresequenz in an sich einfacher Weise herzustellen, die im Organismus zur Bildung von Antikörpern Anlaß geben, die mit dem gesamten Polypeptid des Krankheitserregers bzw. Antigens eine Immunreaktion eingehen. Hierdurch wird es möglich, mit Hilfe eines synthetischen Peptids, das die relevante antigene Determinante eines Krankheitserregers bzw. des pathogenen Mikroorganismus aufweist, Antikörper zu erzeugen, die für die Oberflächenstruktur des betreffenden pathogenen Mikroorganismus spezifisch sind, so daß über diese Antikörper die biologische Aktivität des Krankheitserregers bzw. des pathogenen Mikroorganismus vermindert bzw. blockiert werden kann, so daß eine Immunisierung des mit dem synthetischen Peptid behandelten Organismus erreicht wird.

Die erfindungsgemäße Lehre umfaßt somit die folgenden Teilschritte:

1. Die Erkennung der biologisch, beispielsweise für die Virusneutralisation, relevanten Oberflächenpolypeptide und deren antigene Determinanten,

2. die Sequenzierung dieser relevanten antigenen Determinanten und

3. die Synthese von Peptiden mit der spezifischen Aminosäuresequenz dieser relevanten antigenen Determinanten, welche Peptide dann als Impfstoffe verwendet werden können.

Das erfindungsgemäße Verfahren zur Herstellung des als Impfstoff geeigneten synthetischen Peptids ist nun dadurch gekennzeichnet, daß man

1. einen Krankheitserreger durch eine Detergensbehandlung in einzelne lösliche Strukturpolypeptide zerlegt, die zum Teil solche antigenen Determinanten tragen, die für die Induktion einer Immunität im Wirtsorganismus wesentlich sind,

2. die Strukturpolypeptide mit einem für die relevante antigene Determinante spezifischen monoklonalen Antikörper ausfällt,

3. den gewaschenen Niederschlag fraktioniert,

4. die die relevante Determinante tragende Polypeptidfraktion chemisch und/oder enzymatisch fragmentiert,

5. die gebildeten Fragmente präparativ trennt,

6. die die relevanten antigenen Determinanten tragenden Fragmente mit einem monoklonalen Antikörper
identifiziert,

7. die Aminosäuresequenz des die relevante antigene
Determinante tragenden Fragments in an sich bekannter Weise bestimmt und

8. ein die Aminosäuresequenz der relevanten antigenen
Determinante aufweisendes Peptid in an sich bekannter Weise synthetisiert.

Als Krankheitserreger kann man bei dem erfindungsgemäßen Verfahren Viren, Rickettsien, Bakterien, Mykoplasmen, Pilze, Protozoen und auch Tumorzellen verwenden.
So kann man beispielsweise die Differenzierungsantigene
von Sporozoiten oder Merozoiten von Plasmodium verwenden, um auf diesem Wege eine Immunisierung gegen
Malaria zu bewirken. Es ist auch möglich, das erfindungsgemäße Verfahren auf Kohlehydrat-Determinanten
anzuwenden. Bevorzugt vewendet man jedoch als Krankheitserreger ein Virus oder virale Antigene.

Bei der Detergensbehandlung kann man übliche anionische, kationische oder nichtionische oberflächenaktive Mittel verwenden, wie die unter der Bezeichnung
"Tween" erhältlichen Polyoxyäthylenderivate von Sorbitanestern, die unter der Bezeichnung "Triton" erhältlichen oberflächenaktiven Mittel, Desoxycholat, die
unter der Bezeichnung "Sterox" erhältlichen nichtionogenen oberflächenaktiven Netz- und Reinigungsmittel auf
der Basis von Polyoxyäthylenäthern und -thioäthern,
Natriumdodecylsulfat, die unter der Bezeichnung NP-40 erhältlichen nichtionogenen Tenside auf der Basis von Nonylphenolpolyglycoläthern, Lysolecithin, Octylglycosid und dergleichen.

Die Ausfällung der Strukturpolypeptide in der Stufe 2 des erfindungsgemäßen Verfahren bewirkt man mit einem für die relevante antigene Determinante spezifischen monoklonalen Antikörper, den man durch Klonierung von Zellen erhält, die man aus dem Blut von Rekonvaleszenten oder Immunisierten aus der Lymphflüssigkeit oder durch Punktieren von Lymphknoten gewonnen hat. Es ist weiterhin möglich, auch Milzzellen von Menschen oder Tieren, beispielsweise Mäusen, einzusetzen. Diese monoklonale Antikörper synthetisierenden Zellen können durch klassische Zellklonierungsmethoden, beispielsweise durch Endpunktverdünnung in Mikrotiterplatten gewonnen werden. Vorzugsweise bereitet man sie jedoch unter Anwendung von elektronischen Geräten zur Zellsortierung, da sich hierdurch die Verfahrensweise wesentlich beschleunigen läßt.

Beispielsweise fusioniert man unter Verwendung von Polyäthylenglycol Myelomzellen mit Antikörper produzierenden Zellen. Wenn die verwendete Myelomzelle HATsensitiv ist, so überleben nach Selektion auf einem entsprechenden Kulturmedium nur die Hybridzellen, die das Thymidinkinase-Gen von den Antikörper bildenden Zellen erhalten, welch letztere allein in der Kultur nicht überleben können.

Die danach heranwachsende Zellklone werden mit serologischen Methoden, beispielsweise durch Immunpräzipitation auf die Bildung des gesuchten Antikörpers untersucht.

Die Fraktionierung des gewaschenen Niederschlags in der Stufe 3 des beanspruchten Verfahrens zur Gewinnung der die relevante antigene Determinante tragenden Polypeptidfraktion bewirkt man vorzugsweise durch Chromatographie, durch Zweiphasenaffinitätsverteilung, durch Zweiphasen-Verteilungschromatographie oder durch Gelelektrophorese, wobei man auch eine Affinitätschromatographie mit monoklonalen Antikörpern aus dem Blut von Rekonvaleszenten oder Immunisierten durchführen kann.

Die in dieser Weise gebildete Polypeptidfraktion wird dann chemisch und/oder enzymatisch fragmentiert. Die chemische Fragmentierung kann beispielsweise unter Verwendung von Bromcyan erfolgen, während man bei der enzymatischen Fragmentierung als Enzyme Trypsin, Chymotrypsin, V8-Protease oder dergleichen verwendet.

Die in dieser Weise gebildeten, die relevante antigene Determinante tragenden Fragmente werden präparativ getrennt, beispielsweise durch Hochdruckflüssigkeitschromatographie (HPLC) und/oder durch eine kombinierte Elektrophorese/Dünnschichtchromatographie-Methode und werden dabei mit einem monoklonalen Antikörper, der in der oben beschriebenen Weise gebildet worden ist, identifiziert. Diese präparative Trennung kann unter Anwendung an sich bekannter Verfahrensweisen bewirkt werden, beispielsweise auf das SDS-PAGE mit diskontinuierlichem Puffersystem, durch Chromatographie, gegebenenfalls unter Verwendung von Detergenzien im Puffer (beispielsweise Octylglycosid). Bei der Trennung der Fragmente mit der HPLC ist es auch möglich, die reversed-phase-Technik unter Einsatz eines Acetonitril-Gradienten anzuwenden.

Die Ermittlung der Aminosäuresequenz des in dieser Weise gebildeten Fragments und damit der relevanten antigenen Determinante kann in an sich bekannter Weise erreicht werden, beispielsweise durch schrittweises Abspalten der Aminosäuren (Edman-Abbau) und/oder durch chemische Modifikation einzelner Aminosäuren. Hierbei ist es hilfreich, wenn man bei der Durchführung der erfindungsgemäßen Verfahrens in der ersten Stufe einen Krankheitserreger eingesetzt hat, dessen Polypeptidketten zuvor radioaktiv markiert worden sind. diese radioaktive Markierung kann in vivo durch Zugabe von radioaktiven Aminosäuren zu der Kultur des Krankheitserregers, beispielsweise des Virus erfolgen oder in vitro durch Iodierung, Acylierung, Carboxymethylierung mit entsprechend radioaktiv markierten Reagenzien.

Die bei dem Edman-Abbau freigesetzten einzelnen Aminosäuren können auch als Fluorescamin, Phthaldialdehyd oder Dimethylaminoazobenzolisothiocyanatderivat nachgewiesen werden.

Der Ablauf der Verfahrensschritte des beanspruchten Verfahrens wird dadurch überwacht und verfolgt, daß man die relevante antigene Determinante mit Hilfe des dafür spezifischen monoklonalen Antikörpers nachweist, was durch Blockierung einer Immunpräzipitation des Strukturpolypeptids bzw. einer Virusneutralisation erreicht wird. Diese monoklonalen Antikörper kann man, wie bereits angegeben, dadurch gewinnen, daß man aus dem Blut von Rekonvaleszenten oder gegen das betreffende Antigen immunisierten Organismen gewonnene Zellen kloniert, die die Antikörper gegen die betreffende relevante antigene Determinante bilden. Dieser monoklonale Antikörper wird dann dazu verwendet, die Fraktion bzw. die Fragmente zu identifizieren, die die relevante Determinante tragen. Dies kann beispielsweise durch radioimmuno assay (RIA), durch Enzyme-linked immuno assay (ELISA) oder durch Immunpräzipitation bewirkt werden.

Ein weiterer Gegenstand der Erfindung ist ein Impfstoff der gekennzeichnet ist durch einen Gehalt an einem synthetischen Peptid mit einer Aminosäuresequenz, die relevanten antigenen Determinanten des zu bekämpfenden Krankheitserregers entspricht. Bei der Verabreichung dieses Impfstoffs an den Wirt wird dann nämlich eine entsprechende Immunantwort z.B. Antikörper gegen den Krankheitserreger gebildet und damit eine Immunisierung des Wirtes erreicht.

Das in dem erfindungsgemäßen Impfstoff enthaltene synthethische Peptid besitzt vorzugsweise eine 10 bis 30 Aminosäuren umfassende Aminosäuresequenz. Vorzugsweise enthält der Impfstoff das synthetische Peptid in mit einem Träger gekoppelter Form wozu man natürliche Träger, beispielsweise KLH (Keyhole Limpet Hemocyamin) oder auch künstliche Träger, beispielsweise ein polylysinhaltiges Polypeptid verwenden kann. Ferner kann man auch ein synthetisches oder natürliches Adjuvans einsetzen, daß die Immunreaktion des synthetischen Peptids verstärkt, beispielsweise das inkomplette Freund'sche Adjuvans oder ein trägergekoppeltes Muramyldipeptid.

Im folgenden sei die Erfindung näher erläutert.

Das Blut von Rekonvaleszenten oder immunisierten Personen enthält nicht nur ein Gemisch von Antikörpern gegen verschiedene Determinanten des pathogenen Keimes, sondern auch Antikörper produzierende Zellen, sowie die entsprechenden Vorläufer der B-Zell-Reihe, an deren Ende die Antikörper produzierende Zelle (Plasmazelle) steht. Mit Hilfe an sich bekannter Verfahrensweisen können solche Zellen aus dem peripheren Venenblut zwar angereichert werden, vermögen jedoch in einem Kulturmedium nicht zu überleben. Durch eine Fusionierung mit Myelomzellen, d.h. Antikörper produzierenden Tumorzellen, oder aber durch eine Infektion mit einem Tumorvirus, können die Antikörper produzierenden Zellen aus dem Blut des Rekonvaleszenten bzw. Immunisierten isoliert und zum permanenten Wachstum in einem Kulturmedium gebracht werden. Da eine solche Zelle nur Antikörper gegen eine bestimmte Determinante bildet, können durch Klonierung dieser Zelle homogene Populationen von Hybridzellen gewonnen werden, die nur Antikörper gegen eine spezifische Determinante bilden. Die Herstellung bzw. Gewinnung solcher monoklonaler Antikörper läßt sich erheblich erleichtern und beschleunigen durch die Verwendung von elektronischen Geräten, mit denen die die Antikörper produzierenden Zellen aussortiert werden können. Die von diesen klonierten Zellen erzeugten Antikörper, die auch als monoklonale Antikörper bezeichnet werden, werden anschließend in vitro auf ihre biologische Aktivität (beispielsweise durch Virusneutralisation) oder ihre zytotoxische Wirkung untersucht.

In dieser Weise erhält man monoklonale Antikörper, die für einzelne biologisch relevante Determinanten beispielsweise eines bestimmten Virus, spezifisch wirken, bzw. mit dieser spezifischen antigenen Determinanten tragenden Polypeptide eine Immunreaktion eingehen.

Diese monoklonalen Antikörper werden nun dazu verwendet, in einem Krankheitserreger, beispielsweise einem Viruspräparat ein Polypeptidantigen oder auch eine Kohlehydratstruktur zu erkennen, die die biologisch relevante antigene Determinante trägt. Dies kann durch Immunpräzipitation und nachfolgende Gelelektrophorese erfolgen. Das Polypeptid wird nun durch Chromatographie oder durch präparative Gelelektrophorese isoliert und angereichert. Anschließend wird das isolierte Peptid chemisch und/oder enzymatisch gespalten um Fragménte herzustellen. Diese Fragmente werden dann beispielsweise durch Hochdruckflüssigkeitschromatographie präparativ getrennt, worauf die Fragmente, die die relevante antigene Determinante tragen, durch chemische Reaktion mit dem oben angesprochenen monoklonalen Antikörper identifiziert werden.

Um die Probleme der Zerstörung von Determinanten durch Spaltung zu umgehen, müssen unterschiedliche Fragmentierungsmethoden verwendet werden, um sicher Fragmente zu erhalten, die relevante antigene Determinanten enthalten.

Durch Nachspalten und/oder chemische Modifizierung wird die relevante antigene Determinante auf dem Fragment lokalisiert, anschließend wird die Aminosäuresequenz (die in der Regel 20 bis 30 Aminosäuren umfaßt) des Fragments und damit der relevanten antigenen Determinante unter Anwendung an sich bekannter Methoden (Edman-Abbau etc.) entschlüsselt.

Auf der Grundlage der in dieser Weise ermittelten Aminosäuresequenz wird nun ein synthetisches Polypeptid hergestellt, wozu man an sich bekannte Methoden der Peptidsynthese anwenden kann, vorzugsweise die sogenannte Merrifield Technik oder die Flüssigphasentechnik. In dieser Weise enthält man ein Determinanten-spezifisches Peptid, dessen biologische Wirkung durch Reaktion mit den oben angesprochenen monoklonalen Antikörpern (beispielsweise durch Blockierung der Virusneutralisation oder Immunpräzipitationsreaktion des Strukturpolypeptids) nachgewiesen werden.

Da das in dieser Weise gebildete synthetische Peptid mit der spezifischen Aminosäuresequenz der antigenen Determinante ein relativ niedriges Molekulargewicht besitzt, ist es erfindungsgemäß bevorzugt die immunogene Wirkung dieses Materials durch Kopplung an größere natürliche oder synthetische Trägermoleküle und durch die Verwendung von immunologischen Adjuvantien zu verstärken.

Erfindungsgemäß ist es auch möglich, mehrere verschiedene synthetische Peptide, die die spezifische Aminosäuresequenz antigener Determinanten eines oder mehrerer verschiedener Viren oder anderer Mikroorganismen aufweisen, an den gleichen Träger zu koppeln und für die Immunisierung zu verwenden. Ein in dieser Weise aufgebauter Vielfachimpfstoff ermöglicht es, die Anzahl der zur Immunisierung gegen eine Reihe pathogener Keime notwendigen Impfmaßnahmen wesentlich zu verringern.

0060499

Die beanspruchte Lehre besitzt den besonderen Vorteil, daß mit Ausnahme der Zellzüchtung für die Virusvermehrung und die Herstellung der monoklonalen Antikörper rein chemische Techniken angewandt werden, die sich sowohl automatisieren als auch in technischem Maßstab durchführen lassen. Im Gegensatz zu den technologischen Methoden, die große Zellkulturen benötigen, läßt sich das beanspruchte Verfahren in einfacher Weise bei geringen Kosten durchführen.

Für die Ausführung der oben erörterten Methode sind monoklonale Antikörper überlegen. Es können aber auch spezifische Serumantikörper angewendet werden.

PATENTANSPRÜCHE

1. Verfahren zur Herstellung eines als Impfstoff
geeigneten synthetischen Peptids, d a d u r c h
g e k e n n z e i c h n e t , daß man

1) einen Krankheitserreger durch eine Detergensbehandlung in einzelne lösliche Strukturpolypeptide zerlegt, die zum Teil relevante
antigene Determinanten tragen, die für die
Induktion einer Immunität im Wirtsorganismus
wesentlich sind,

2) die Strukturpolypeptide mit einem für die
relevante antigene Determinante spezifischen
monoklonalen Antikörper ausfällt,

3) den gewaschenen Niederschlag fraktioniert,

4) die die relevante antigene Determinante
tragende Polypeptidfraktion chemisch und/oder
enzymatisch fragmentiert,

5) die gebildeten Fragmente präparativ trennt,

6) die die relevanten antigenen Determinanten
tragenden Fragmente mit einem monoklonalen
Antikörper identifiziert,

7) die Aminosäuresequenz des die relevante
antigene Determinante tragenden Fragments in
an sich bekannter Weise bestimmt und

8) ein die Aminosäuresequenz der relevanten antigenen Determinante aufweisendes Peptid in an sich bekannter Weise synthetisiert.

2. Verfahren nach Anspruch 1, d a d u r c h  g e - k e n n z e i c h n e t ,  daß man die Fraktionierung des gewaschenen Niederschlags der Stufe 3 durch Gelelektrophorese, durch Zweiphasenaffinitätsverteilung und/oder durch Zweiphasen-Verteilungschromatographie bewirkt.

3. Verfahren nach den Ansprüchen 1 oder 2, d a - d u r c h  g e k e n n z e i c h n e t ,  daß man als Krankheitserreger, Viren, Rickettsien, Bakterien, Mykoplasmen, Pilze, Protozoen oder Tumorzellen verwendet.

4. Verfahren nach Anspruch 3, d a d u r c h  g e - k e n n z e i c h n e t ,  daß man einen in seinen Polypeptidketten radioaktiv markierten Krankheitserreger verwendet.

5. Verfahren nach den Ansprüchen 1 bis 4, d a - d u r c h  g e k e n n z e i c h e t ,  daß man die Gel- elektrophorese auf einem Polyacrylamidgel durch- führt.

6. Verfahren nach den Ansprüchen 1 bis 5, d a - d u r c h  g e k e n n z e i c h e t ,  daß man die rele- vante antigene Determinanten tragenden Polypeptide durch Chromatographie oder durch Gelelektrophorese präpariert.

7. Verfahren nach den Ansprüchen 1 bis 6, d a -
d u r c h  g e k e n n z e i c h n e t , daß man das die
relevante antigene Determinante tragende Fragment
durch Hochdruckflüssigkeitschromatographie
und/oder durch kombinierte Elektrophorese und
Dünnschichtchromatographie abtrennt.

8. Verfahren nach den Ansprüchen 1 bis 7, d a -
d u r c h  g e k e n n z e i c h n e t , daß man die
Aminosäuresequenz des die relevante antigene
Determinante tragenden Fragments und damit der
relevanten antigenen Determinante durch Spaltung
und/oder chemische Modifikation einzelner Aminosäuren bestimmt.

9. Verfahren nach den Ansprüchen 1 bis 8, d a -
d u r c h  g e k e n n z e i c h n e t , daß man den
Ablauf der Verfahrensschritte durch Nachweis der
relevanten antigenen Determinante mit Hilfe des
dafür spezifischen monoklonalen Antikörpers überwacht und verfolgt.

10. Verfahren nach Anspruch 9, d a d u r c h  g e -
k e n n z e i c h n e t , daß man monoklonale Antikörper verwendet, die man durch Klonierung von
Zellen gebildet hat, die man aus dem Blut von
Rekonvaleszenten oder Immunisierten, aus der
Lymphflüssigkeit, durch Punktieren von Lymphknoten
oder aus Milzzellen gewonnen hat.

11. Verfahren nach Anspruch 10, d a d u r c h  g e -
k e n n z e i c h n e t , daß man monoklonale Antikörper einsetzt, die man durch Endpunktverdünnung in Mikrotiterplatten und/oder unter Verwendung von elektronischen Geräten zur Zellensortierung gewonnen hat.

12. Impfstoff, gekennzeichnet durch einen Gehalt an einem synthetischen Peptid mit einer der antigenen Determinante eines Krankheitserregers entsprechenden Aminosäuresequenz.

13. Impfstoff nach Anspruch 12, dadurch gekennzeichnet, daß das synthetische Peptid eine 10 bis 30 Aminosäuren umfassende Aminosäuresequenz aufweist.

14. Impfstoff nach den Ansprüchen 12 oder 13, dadurch gekennzeichnet, daß er das synthetische Peptid in mit einem Träger gekoppelter Form enthält.

15. Impfstoff nach Anspruch 14, dadurch gekennzeichnet, daß er als Träger ein synthetisches oder natürliches, die Immunreaktion verstärkendes Adjuvans enthält.

16. Impfstoff nach den Ansprüchen 12 bis 15, dadurch gekennzeichnet, daß er aus einem gemäß den Ansprüchen 1 bis 11 erhältlichen synthetischen Peptid als Wirkstoff und gegebenenfalls an sich bekannten Adjuvantien, Bindemitteln, Trägermaterialien und/oder weiteren Hilfsstoffen besteht.